(19) Europäisches Patentamt
European Patent Office
Office européen des brevets

(11) Publication number: 0 209 611 A1

(12) EUROPEAN PATENT APPLICATION

(21) Application number: 85108997.9

(51) Int. Cl.4: C25C 3/04

(22) Date of filing: 18.07.85

A request for correction of the description has been filed pursuant to Rule 88 EPC. A decision on the request will be taken during the proceedings before the Examining Division (Guidelines for Examination in the EPO, A-V, 2.2).

(43) Date of publication of application: 28.01.87 Bulletin 87/05

(84) Designated Contracting States: BE DE FR GB IT NL

(71) Applicant: THE DOW CHEMICAL COMPANY
2030 Dow Center Abbott Road P.O. Box 1967
Midland, MI 48640(US)

(72) Inventor: Kyriacou, Demetrios
1035 Silver Avenue
San Francisco, Cal. 94 134(US)
Inventor: Brattesani, Donald Neil
816 Grosvenor Place
Oakland, Cal. 94610(US)

(74) Representative: Weickmann, Heinrich, Dipl.-Ing. et al
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820
D-8000 München 86(DE)

# (54) Electrolytic cell comprising stainless steel anode and a process for preparing polychloropicolinate anions.

(57) An important increase in the yield of 3,6-dichloropicolinic acid prepared by electrolytic reduction of tetrachloro-2-picolinic acid (in basic, aqueous solution, at a silver cathode) results when a stainless steel anode is used, rather than those (graphite, e.g.) taught in the prior art.

EP 0 209 611 A1

# ELECTROLYTIC CELL COMPRISING STAINLESS STEEL ANODE AND A PROCESS FOR PREPARING POLYCHLOROPICOLINATE ANIONS

Production of 3,6-dichloropicolinic acid (3,6-D), a highly active plant growth regulator, by electrolytic reduction of tetrachloro-2-picolinic acid ("tet-acid") at a silver cathode in basic, aqueous solution is disclosed in U.S.P. 4,217,185.

The cathode has a surface layer of silver microcrystals formed by the electrolytic reduction of colloidal, hydrous, silver oxide particles in the presence of an aqueous base. The presence of other noble metals is not beneficial and the presence of base metals (nickel and copper, most notably) definitely lowers the activity of the cathode. However, other conductive metals, including stainless steel, may serve as a substrate on which the silver layer is formed, provided that the substrate metal not be subjected to anodization (as in a preferred method of forming the precursor silver oxide particles).

Inert electrode materials are generally suitable for the anode to be used in conjunction with the silver cathode, but in order to attain 3,6-D yields of 90 percent or more, it is necessary, according to the patent, to employ anodes consisting essentially of graphite. Other anode materials are believed to encourage decarboxylation ("Kolbe type" oxidation of polychloropyridine carboxylate anions).

It has been found that 3,6-D yield and purity are sensitive to the type of graphite the anode is made of. Furthermore, even the most suitable graphite found (Union Carbide grade ATL graphite) undergoes spalling and a drop-off in 3,6-D yield and purity is experienced as the anode ages in use. This is readily corrected by replacing the anode with a fresh one, but that results in higher capital and operating expenses. Accordingly, it is apparent that an otherwise suitable anode material less susceptible to deleterious alterations in prolonged use would be highly desirable.

This invention provides a superior anode for use with a cathode at which tetrachloro-2-picolinic acid in aqueous, basic solution can be selectively reduced to 3,6-dichloropicolinic acid in high yield.

The invention also provides an electrolytic cell incorporating the improved anode and adapted for the conversion of the tet-acid to 3,6-D.

The invention further provides a means of avoiding the costs and processing interruptions associated with periodic replacement of graphite anodes (and the consequent fluctuations in the purity of the 3,6-D product) and a means to eliminate the source of the trace metal impurities known to be present in available graphites -which impurities are believed responsible for the apparent need for periodic acid washing (of the cathode) disclosed in the prior act.

The cell of the invention comprises a stainless steel anode and a cathode at which tetrachloro-2-picolinate anions in basic, aqueous solution can be selectively reduced in high yield to 3,6-dichloro-2-picolinate anions.

The electrolyte employed in the cell is a working component of the cell, not the material worked on (reduced or oxidized) by the cell. In those instances in which electrolysis of water (formation of hydrogen at the cathode and/or of oxygen at the anode) occurs as a side or co-reaction, the unconverted portion of the aqueous base still functions as the electrolyte.

The process of the invention is the use of the foregoing cell in the electrolytic process for the coproduction of oxygen and polychloropicolinate anions of the structure

(A) 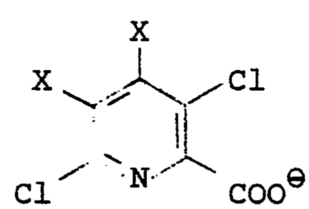

wherein one X is H and the other is H or Cl, which comprises

providing a solution in an aqueous base of a polychloropicolinic acid of the structure

(B) 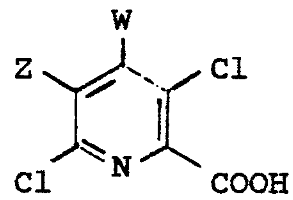

wherein both Z and W are Cl, or one is Cl and the other is H,

and, while agitating said solution, passing an electric current therethrough from the anode to said cathode,

the solution having a temperature within the range of from 5° to 60°C, a pH of about 13 or more and containing at least 0.08 hydroxyl ions per chloride ion present therein,

the cathode having a potential, relative to a saturated calomel reference electrode, of from -0.8 to -1.8 volts and the anode having a potential, relative to the cathode, such that the density of the current is from 0.005 to 0.085 amperes per $cm^2$ of projected cathode surface,

thereby forming anions of said polychloropicolinic acid (A) at the cathode and oxygen at the anode.

The process of the present invention is the improvement which comprises employing stainless steel as the anode material.

Suitable anode materials for the practice of the present invention are those alloys of chromium, nickel and iron -commonly designated as stainless steels -which are highly resistant to corrosion by basic, aqueous chloride brines at temperatures up to 60°C. (The resistance to corrosion by basic brines at various $OH^-$ and $Cl^-$ concentrations is well known for a wide range of stainless steel compositions.) The presently most preferred such material is 316 stainless steel (which has the composition 0.11 weight percent C, 17-19 weight percent Cr, 7-11 weight percent Ni and 2-4 weight percent Mo (the balance consisting essentially of Fe). Other specific suitable stainless steels are grades 302 through 310 and 320, 321 and 403.

The preferred cathode for the practice of the present invention is one having a surface layer of silver microcrystals formed by the electrolytic reduction of colloidal, hydrous silver oxide particles in the presence of an aqueous base. However, any other cathode capable of selectively reducing tetrachloro-2-picolinate anions to 3,6-dichloro-2-picolinate anions in yields of at least 90 mole percent may be employed with the stainless steel anode.

Polychloropicolinic acids of the preceding formula (B) may be reduced to the corresponding acids of formula (A) as known in the art but employing the cell of the present invention, i.e., a cell comprising a stainless steel anode, a basic aqueous electrolyte and a cathode having the capability specified in the immediately preceding paragraph.

The electrodes in the latter cell may be of any configuration, the same or different. However, it is preferred that the cathode and anode take the form of concentrically disposed cylinders.

. Similarly, each electrode may be formed of a solid bar or sheet, a screen or "expanded" sheet. High surface area forms, such as screens or other foraminous sheets, for example, are preferred, particularly in those applications in which the cell contents are agitated.

It should be noted that the anode does not have to be a monolithic body of metal. It is only necessary that those portions of the anode surface layer subject to contact with the cell contents consist of stainless steel (and that the anode as a whole is otherwise suitable).

The utility of the present cell of course is not limited to reductions of polychloropicolinic acids. It may be employed for the reduction (or oxidations) of any substrate material which is soluble in an aqueous base and does not (as such or in its reduced or oxidized form) detrimentally react with the electrolyte to an intolerable extent. Where appropriate, the cell may also comprise a diaphragm (or an equivalent anolyte/catholyte separating means) and/or agitation of the cell contents may be dispensed with. Such other modifications, or ancillary apparatus as may be necessary or appropriate to utilization of the present cell in any particular electrochemical application will be apparent to those skilled in the art of electrochemistry. Similarly, the appropriate values for such process parameters as temperature, electrode potentials, pH, choice of base, base concentration, etc., will also be apparent to those skilled in the art.

Any otherwise suitable source of hydroxyl and counter ions may be employed as the base in the cell (and process) of the present invention. However, alkali metal hydroxides are preferred, most notably KOH or NaOH, the latter being particularly preferred.

The term "otherwise suitable" of course implies that the base employed does not detrimentally react with any component of the cell itself or any other component of the cell contents to an intolerable degree.

A restraint is imposed on base concentration by the susceptibility of at least some stainless steels to corrosion at low hydroxide concentrations. In the case of 316 stainless, the hydroxide concentration should not be allowed to drop below about 1.0 weight percent, particularly at temperatures above normal ambient temperatures.

It should be noted that the term "aqueous base" is not intended to exclude the presence in the anolyte and/or catholyte of other substances, such as, for example, organic co-solvents or soluble salts, which do not detrimentally affect obtention of the desired product.

The following examples further illustrate the invention.

Example 1

To a 200 ml electrolytic beaker equipped with a Teflon®-coated magnetic stirring bar, a cylindrical silver screen cathode, a cylindrical, imperforate 316SS anode, a Luggin capillary tube fitted with a standard calomel electrode (SCE) and a thermometer, was added enough 1/1, v/v, conc. $HCl/H_2O$ to fill the cell (Luggin capillary removed). The aqueous HCl was stirred in the cell for 10 min. followed by draining, rinsing well with reverse osmosis purified (RO) water, then filling with 108.24 gms. of 7.0 weight percent NaOH (mercury grade caustic; solution prepared with RO water). The cathode was anodized to +0.6V vs SCE for 5 min. (3 amps maximum), followed by cathodization to -1.3V vs SCE (3 amps maximum), giving a background current of 0.5 amperes. The tet acid (11.76 g, 0.0451 mols) was added portion-wise over 3.5 hours by masticating each 2 gm portion with cell liquor, then returning the resulting slurry to the bulk of the solution. The electrolysis proceeded as follows (the cathode potential relative to the SCE remaining at -1.3 volts throughout, except during periodic anodization intervals as noted) for a total reaction time of 5.4 hours (382-57=325 minutes)):

TABLE I

| Elapsed Time (Minutes) | Temp. (°C.) | Cell Current Amps | Comments |
|---|---|---|---|
| 0 | 20.5 | - | 1/1, v/v, conc. HCl/$H_2O$ added for cleansing. |
| 10 | 20.0 | - | Drained aq. HCl, rinsed well w/R.O. $H_2O$; 7.0% NaOH added. |
| 16 | 20.0 | - | Anodization: +0.6v/5 min./0.35 amps final. |
| 56 | 22.0 | 1.1 | Anodization complete. |
| 57 | 22.0 | 0.5 | Adding 2 gms TA.[1] |
| 62 | 23.5 | 4.3 | Addition complete. |
| 87 | 23.5 | 1.1 | Adding 2 gms TA. |
| 91 | 25.0 | 4.2 | Addition complete. |
| 117 | 25.0 | 1.3 | Adding 2 gms TA. |
| 120 | 26.0 | 4.2 | Addition complete. |
| 147 | 26.0 | 1.2 | Anodization: +0.6v/9 min./0.48 amps final. |
| 174 | 26.0 | 2.1 | Anodization complete. |
| 177 | 26.0 | 1.0 | Adding 2 gms TA. |
| 180 | 27.5 | 4.6 | Addition complete. $E_{cell}$ = 2.4v. |
| 207 | 27.0 | 0.8 | Adding 2 gms TA. |
| 210 | 28.5 | 4.7 | Addition complete. |
| 237 | 28.0 | 0.9 | Anodization: +0.6v/8 min./0.48 amps final. |
| 263 | 28.0 | 1.6 | Anodization complete. |
| 267 | 28.0 | 0.8 | Adding 0.6 gm TA. |
| 269 | 28.0 | 2.3 | All TA added. |
| 327 | 26.5 | 0.4 | Anodization: +0.6v/9 min./0.7 amps final. |
| 352 | 27.0 | 1.4 | Anodization complete. |
| 382 | 25.5 | 0.48 | STOP; suspended solid in cell liquor. |

Note: 1. tet-acid.

The cell liquor was filtered by suction through celite, then stored in the cold (5°C.) for 65 hours. The cell liquor (pH 12.56) was acidified with conc. HCl to pH 1.00, and the resulting mixture was extracted with $CH_2Cl_2$ (3×100 ml; 9×50 ml). The combined organic layer was dried over anhydrous $Na_2SO_4$ (5 min.), filtered, and the solvent was removed at reduced pressure (rotary evaporator, 50°C., 30 min.) to give 8.65 gms of white crystalline solid.

The latter solid was found (by Gas/Liquid Phase Chromatography) to be 98.458 weight percent 3,6-dichloro-2-picolinic acid. The theoretical yield of 100 percent 3,6-D was calculated as $(0.98458 \times 8.65) \div (191.96 \times 0.04508) = 98.4$ percent.

Example 2 (Comparison of SS and graphite anodes)

In essentially the manner of Example 1, a series of runs was carried out using three different grade graphite anodes and one grade (316) stainless steel anode. The results are given in Table II below:

TABLE II

The Effect of Different Anode Materials

| Run | Anode[2] | 3,6-D Yield (%) | PRODUCT DISTRIBUTION (WT. %)[1] 3,6-D | MCPA | 4,5-DCPA | CPA | Tet-Acid Accountability[3] |
|---|---|---|---|---|---|---|---|
| 1 | ATL | 93.8 | 98.0 | 0.8 | 0.6 | - | 99.4 |
| 2 | ATL | 97.0 | 99.0 | 1.1 | 0.4 | - | 100.5 |
| 3 | ATL | 97.0 | 99.0 | 1.1 | 0.5 | - | 100.6 |
| 4 | ATL | 95.0 | 98.3 | 1.2 | 0.7 | - | 100.2 |
| 5 | ATL | 96.5 | 99.2 | 1.0 | 0.5 | - | 100.8 |
| 6 | ATL | 96.0 | 98.6 | 1.0 | 0.7 | - | 100.3 |
| 7 | ATL | 95.7 | 98.3 | 1.0 | 0.5 | - | 99.8 |
| 8 | AGSR | 67.6 | 74.0 | 2.7 | 1.9 | 17.2 | 95.8 |
| 9 | ECV | 57.0 | 73.7 | 4.5 | 2.6 | 13.2 | 94.3 |

TABLE II (continued)

The Effect of Different Anode Materials

| Run | Anode[2] | 3,6-D Yield (%) | PRODUCT DISTRIBUTION (WT. %)[1] 3,6-D | MCPA | 4,5-DCPA | TCPA | Tet-Acid Accountability[3] |
|---|---|---|---|---|---|---|---|
| 10 | 316SS | 96.5 | 98.0 | 2.3 | 0.7 | - | 101.0 |
| 11 | 316SS | 97.5 | 98.6 | 2.2 | 0.6 | - | 101.4 |
| 12 | 316SS | 98.4 | 98.5 | 1.6 | 0.5 | - | 100.6 |
| 13 | 316SS | 100.0 | 98.1 | 1.6 | 0.8 | - | 100.5 |
| 14 | 316SS | 99.7 | 98.4 | 1.4 | 0.9 | - | 100.7 |
| 15 | 316SS | 100.0 | 99.0 | 1.0 | 1.0 | - | 101.0 |
| 16 | 316SS | 99.4 | 98.0 | 0.9 | 0.8 | - | 99.7 |
| 17 | 316SS | 99.4 | 98.0 | 1.0 | 0.8 | - | 99.8 |
| 18 | 316SS | 99.8 | 98.6 | 1.6 | 0.5 | - | 100.7 |
| 19 | 316SS | 99.6 | 98.0 | 1.3 | 0.5 | - | 99.7 |
| 20 | 316SS | 99.3 | 98.0 | 1.3 | 1.0 | - | 100.3 |
| 21 | 316SS | 97.7 | 98.0 | 1.3 | 0.8 | - | 100.1 |

Notes:
1. 3,6-D = 3,6-dichloropicolinic acid; 4,5-DCPA = 4,5-dichloropicolinic acid; MCPA = monochloropicolinic acid; TCPA = trichloropicolinic acids.
2. ATL = medium priced general purpose graphite with consistent medium grain size.
   AGSR = economical, general purpose graphite of variable grain size.
   ECV = graphite for high purity applications possessing small grain size; ash level of ca. 50 ppm.
   316SS = stainless steel grade 316.
   ATL, AGSR, and ECV are graphite materials produced by the Union Carbide Corporation, Chicago, Ill.
3. % of starting material accounted for by products.

It will be seen that the yield of 3,6-D (of essentially the same purity) was consistently higher with the stainless steel anode. The average yield for the seven runs with (fresh) ATL graphite anodes was 95.86 percent and the average for the twelve runs with the (same) stainless steel anode was 98.94 percent. Ignoring the very minor difference in average accountability for the two series, at least a 3 percent higher yield is indicated for the stainless steel anode. For a process operated on a commercial scale, this is an important improvement.

When the same ATL graphite anode was used for thirteen successive runs, a drop in product yield and purity to 83 percent of 91 percent pure 3,6-D resulted. (A run made with the same cell but with a fresh ATL anode resulted in a 99 percent yield of 99 percent pure 3,6-D.) In contrast, a 97.7 percent yield of 98 percent pure 3,6-D was obtained from run number 12 (Table II) with the stainless steel anode.

## Claims

1. An electrolytic cell characterized by having a stainless steel anode and a cathode having a surface layer of silver microcrystals formed by electrolytic reduction of colloidal, hydrous silver oxide particles in the presence of an aqueous base.

2. Cell of Claim 1 characterized in that the anode and cathode are of a cylindrical configuration and one is disposed concentrically within the other.

3. Cell of Claim 2 characterized in that least one of the anode or cathode is in the form of a foraminous sheet.

4. Cell of Claim 3 characterized in that the sheet is a screen.

5. Cell of Claim 1 characterized by containing a basic, aqueous electrolyte.

6. Process for the co-production of oxygen and polychloropicolinate anions of the structure

(A)

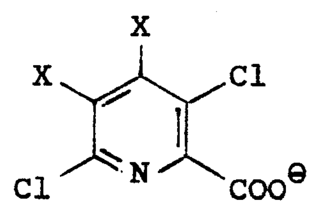

wherein one X is H and the other is H or Cl,

by providing a solution in an aqueous base of a polychloropicolinic acid of the structure

(B)

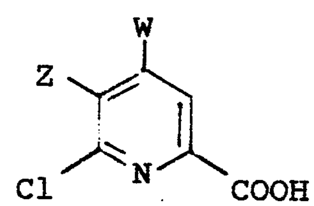

wherein both Z and W are Cl or one is Cl and the other is H,

and, while agitating said solution, passing an electric current therethrough from an anode to a cathode,

the solution having a temperature within the range of from 5° to 60°C, a pH of about 13 or more and containing at least 0.08 hydroxyl ions per $Cl^-$ ion present therein,

the cathode

(a) being one at which tetrachloro-2-picolinate anions in basic, aqueous solution can be reduced to 3,6-dichloro-2-picolinate anions, and

(b) having a potential relative to a saturated calomel electrode of from -0.8 to -1.8 volts,

characterized in that the anode is a stainless steel anode.

7. Process of Claim 6 characterized in that the anode has a potential, relative to the cathode, such that the density of the current is from 0.005 to 0.085 amperes per $cm^2$ of projected cathode surface, thereby forming anions of said polychloropicolinic acid (A) at the cathode and oxygen at the anode.

European Patent Office

# EUROPEAN SEARCH REPORT

Application number

EP 85 10 8997

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 497 697 (MARSHALL et al.) * Column 3, lines 41-54; example 1; table 3 * | 1,5-7 | C 25 C 3/04 |
| Y | | 2-4 | |
| | --- | | |
| Y | EP-A-0 018 069 (THE DOW CHEMICAL COMP.) * Page 8, line 30 - page 9, line 1; example 1; page 24, lines 12-17 * | 2-4 | |
| | --- | | |
| Y,D | US-A-4 217 185 (KYRIACOU et al.) * Column 9, lines 31-34; column 13, lines 46-56; claims 1,15 * | 1-7 | TECHNICAL FIELDS SEARCHED (Int. Cl.4) |
| | --- | | |
| Y | US-A-2 925 371 (VAN WINCKEL) * Column 2, lines 51-54 * | 1-7 | C 25 C |
| | --- | | |
| A | US-A-4 460 441 (DOMNING) * Claims 1,3,4 * | 1-7 | |
| | --- | | |
| E | US-A-4 533 454 (KYRIACOU et al.) * Whole document * | 1-7 | |
| | ----- | | |

The present search report has been drawn up for all claims

| Place of search | Date of completion of the search | Examiner |
|---|---|---|
| THE HAGUE | 13-03-1986 | COOK S.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

17

PATENTANWÄLTE DIPL.-ING. H. WEICKMANN, DIPL.-PHYS. DR. K. FINCKE
DIPL.-ING. F. A. WEICKMANN, DIPL.-CHEM. B. HUBER
DR.-ING. H. LISKA, DIPL.-PHYS. DR. J. PRECHTEL

EPA-EPO-OEB
DG 1
Rec
28 08 1985

8000 MÜNCHEN 86
POSTFACH 860 820
MÜHLSTRASSE 22
TELEFON (0 89) 98 03 52
TELEX 5 22 621
TELEGRAMM PATENTWEICKMANN MÜNCHEN

HMW

European Patent Application
No. 85 108 997.9
The Dow Chemical Company
Dow Case 29,338-F - EU 647

Attached hereto is a new page 15 on which formula (B) has been brought into correspondence with formula (B) indicated at page 4. An obvious typographical error is concerned here, the correction of which is, in our view, admissible pursuant to Rule 41 and does not constitute an amendment pursuant to Rule 86.

EPA-EPO-OEB
MÜNCHEN
Empfang bestätigt
Receipt acknowledged
Accusé reception

Encl.:
new page 15,
in triplicate

To the
European Patent Office
8000 Munich 2